# EUROPEAN PATENT APPLICATION

(11) **EP 4 687 093 A1**
(43) Date of publication of application: **04.02.2026**
(21) Application number: 24306300.5
(22) Date of filing: 01.08.2024
(51) Int. Cl.: G06T 3/14, A61B 6/50, G06T 7/00

(54) **REMOVING LEADS INSIDE A VESSEL**

(71) Applicant: Koninklijke Philips N.V., 5656 AG Eindhoven (NL)
(72) Inventor: HENDRIKS, Bernardus, 5656 Eindhoven (NL); FLORENT, Raoul, 5656 Eindhoven (NL); ELENBAAS, Thijs, 5656 Eindhoven (NL)
(74) Representative: Philips Intellectual Property & Standards

(57) **Abstract**

The present invention relates to the field of removing leads inside a vessel. For an improved imaging during an extraction procedure, a device (10) for determining an adhesion of a lead inside a vessel is provided. The device comprises a data input (12), a data processor (14) and an output interface (16). The data input is configured to provide a plurality of images of a region of interest of a blood vessel of a subject, in which region of interest a lead is located inside the vessel. The data processor is configured to align at least a part of the images of the plurality of images with each other. The aligning is provided based on a predetermined feature in the image relating to a spatial relation of the lead in the vessel. The data processor is also configured to stack the aligned images. Further, the output interface is configured to provide the stacked images for assessment of an adhesion of the lead at an inner side of the vessel wall.

## Description

### FIELD OF THE INVENTION

The present invention relates to the field of removing leads inside a vessel. The present invention relates in particular to a device for determining an adhesion of a lead inside a vessel, to a system for determining an adhesion of a lead inside a vessel and to a method for determining an adhesion of a lead inside a vessel

### BACKGROUND OF THE INVENTION

Implanted medical devices are used for many purposes. As an example, pacemakers are medical devices that are implanted in patients with a slow heart rate to help regulate their heartbeat. Over time, these devices can become infected, damaged, or fail for other reasons, which may require their removal. The process of removing a pacemaker is called lead extraction. Lead extraction involves the removal of the wires, or leads, that connect the pacemaker to the heart. This can be a complex and crucial procedure, as the leads can become tightly embedded in the surrounding tissue or even the heart muscle itself. In order to remove them, specialized tools and techniques are used, such as laser sheaths, snares, and mechanical extractors. The procedure can be performed in a catheterization laboratory under X-ray guidance, e.g. fluoroscopy or venography imaging. Lead extraction is generally considered a safe and effective procedure. It is estimated that 5% of leads will be extracted over their implanted lifespan. However, lead extraction can become a demanding task; especially extractions of a lead in the super vena cava are difficult due to the curved nature and the serious complications that can arise. Knowledge about the leads and their location within a vessel is considered important. In particular the adhesion of the lead to the blood vessel plays an important role for the lead extraction. As an example, WO 2022/128703 A1 determines whether a section of a lead has adhered to a blood vessel. However, it has been shown that a demand exists for a more precise determination of the adherence.

### SUMMARY OF THE INVENTION

There may thus be a need for improved imaging during an extraction procedure.

The object of the present invention is solved by the subject-matter of the independent claims; further embodiments are incorporated in the dependent claims. It should be noted that the following described aspects of the invention apply also for the device for determining an adhesion of a lead inside a vessel, for the system for determining an adhesion of a lead inside a vessel and for the method for determining an adhesion of a lead inside a vessel.

According to the present invention, a device for determining an adhesion of a lead inside a vessel is provided. The device comprises a data input, a data processor and an output interface. The data input is configured to provide a plurality of images of a region of interest of a blood vessel of a subject, in which region of interest a lead is located inside the vessel. The data processor is configured to align at least a part of the images of the plurality of images with each other. The aligning is provided based on a predetermined feature in the image relating to a spatial relation of the lead in the vessel; and to stack the aligned images. The output interface is configured to provide the stacked images for assessment of an adhesion of the lead at an inner side of the vessel wall.

As a result, a boost effect allows a facilitated identification of relatively moving parts and relatively non-moving parts. The aligned stacking is focused e.g. on either the vessel wall or a part of the lead. If the lead is somehow fixed to the vessel wall, no relative motion between wall and lead will take place and that will increase the visible structures like in a boosting action. If the lead is not fixed to the vessel wall, relative motion between wall and lead will take place and that motion will lead to blur when stacking the images. The result will be easy to identify by assessment of the signal-to-noise ratio or even by visual inspection.

According to an example, after the alignment, an increase of a signal-to-noise ratio for parts of the image other than the predetermined feature indicates a static relation of the respective part of the image and the predetermined feature.

According to an example, the data processor is configured to provide the aligning with respect to a wall section of the vessel. As an option, the wall section of the vessel for alignment is in a region of the vessel around the lead. As a further option, after the alignment, an increase of a signal-to-noise ratio for a part of the lead indicates a static relation of the lead and the vessel wall.

According to an example, the data processor is configured to provide the aligning with respect to a section of the lead in the region of interest. As an option, the section of the lead is in a region of the vessel where the lead is closer to the vessel wall. As a further option, after the alignment, an increase of a signal-to-noise ratio for a part of the vessel wall adjacent to the lead indicates a static relation of the vessel wall and the lead.

According to an example, the data processor is configured to mark image parts indicating the static relation, after the stacking.

According to an example, for the assessment of the adhesion, the data processor is configured to identify a signal-to-noise ratio. As an option, for the assessment of the adhesion, the data processor is configured i) to identify lead sections in the images and to assess a respective signal-to-noise ratio for the lead sections. Alternatively or in addition, the data processor is configured ii) to identify vessel sections in the images and to assess a respective signal-to-noise ratio for the vessel sections.

According to an example, the data processor is configured to identify, as a base, a signal-to-noise ratio for a first part of the vessel. The data processor is also configured to identify a signal-to-noise ratio for further parts of the vessel that are linked to the lead inside the vessel. For the identification of the signal-to-noise ratio, the first part acts as a base.

According to an example, the data input is configured to provide a predetermined threshold of the signal-to-noise ratio to differentiate moving and non-moving parts of the lead. The data processor is configured to label the non-moving parts as adhered lead portions.

According to an example, the data processor is configured to provide the aligning and the stacking for a selection-part of the vessel. The selection-part is movable along the vessel to provide a repeated aligning and stacking along the vessel structure.

According to an example, the images are X-ray images. As an option, at least a part of the images shows an at least partly contrast-injected vasculature.

According to the present invention, also a system for determining an adhesion of a lead inside a vessel is provided. The system comprises an imaging device and a device for determining an adhesion of a lead inside a vessel according to one of the preceding examples. The imaging device is configured to provide the plurality of images of region of interest of a blood vessel of a subject.

According to the present invention, also a method for determining an adhesion of a lead inside a vessel is provided. The method comprises the following steps:
- Providing a plurality of images of a region of interest of a blood vessel, in which region of interest a lead is located inside the vessel;
- Aligning at least a part of the images of the plurality of images with each other; wherein the aligning is provided based on a predetermined feature in the image relating to a spatial relation of the lead in the vessel;
- Stacking the aligned images; and
- Providing the stacked images for assessment of an adhesion of the lead at an inner side of the vessel wall.

According to an aspect, a sequence of images showing a lead in a vessel structure is aligned to a defined structure, and then stacked in order to provide a blurring effect for relatively moving parts and an intensifying effect for relatively non-moving parts. The sequence of images shows a vascular structure during heartbeat motion leading to movement of the vessel and the enclosed lead part. The stacking results in a modification of the signal-to-noise ratio of the achieved "combined" image. The signal-to-noise ratio is determined to assess whether a relative movement is present or not. The detection of the signal-to-noise ratio allows an assessment of the adherence of the lead inside the vessel.

According to an aspect, a series of X-ray images of the blood vessel with the lead inside is obtained, for example as a venogram. The X-ray images are stacked after aligning them with respect to the blood vessel wall. Since the vessel is likely too long to register and to stack as a whole, a sub-selection is made, for example, of an area of the vessel close to the relevant part of the lead.

As a result, for the static structures with respect to the vessel wall, the signal-to-noise of these pixels of these structures increases, while for non-static structures, this would not increase and rather decrease. When the S/N increases for certain parts of the lead, this would indicate that this lead part is most likely stuck to the vessel wall. These areas may then be marked in the image and presented to the physician.

In an example, an X-ray system is provided, comprising an X-ray source-detector system on a C-arm geometry. The C-arm is moved by a motorized geometry allowing the interior of the patient to be imaged. Furthermore, the room is equipped with a patient table, also referred to as subject support, monitors to show the X-ray images to the physician and a control room allowing the system to be controlled outside the room.

In an example, venography is provided as a medical imaging procedure that uses X-ray radiation to visualize the blood vessels in the body, particularly the veins. The technique can involve injecting a contrast material, or dye, into the vessel, e.g. the veins, which makes the vessel visible on X-ray images.

In an example, the X-ray images, e.g. a venogram, are aligned with respect to the vessel wall and "stacked". For example, the pixel intensities of the images are added together after aligning. Hence, moving structures and noise will be smeared out, while static images will increase in signal-to-noise ratio.

In an option, the venogram images are undergoing the following process:
- The images are filtered in order to remove or dim device contributions. This can be done using computer vision filtering techniques that attenuate device frequencies such as sharp ridges. But other inpainting techniques can also be used, based or not on deep learning approaches.
- Further, likewise, vessel contributions are enhanced, using some form of vesselness filter. Vessels are viewed as large absorbing and elongated areas and as such can be enhanced compared to the surrounding background. Exact segmentation, which might be difficult, is not necessary.
- On the processed images where devices are dimmed and vessels are enhanced, a motion field can be computed that will mainly focus on the vessel walls.

In an option, as the approximate region of interest, i.e. a region where the lead is connected to the vessel wall, is known, but not precisely, the images are stacked based on the motion field in that region, rather than the full vasculature.
- In an option, affine transformations are involved.
- In another option, non-affine transformations are involved.
- Next, the computed vector field that will link any injected image of the run to a reference injected image will be used to register the full injected image set to this fixed reference. As an example, it is resorted to the identification of the contrast-injected images via the processing of the injection curve, e.g. integral of a certain metric along the time axis.
- Once registered, the images, e.g. where the devices' appearance has been restored, can be correctly aligned with respect to the chosen reference and then temporally integrated. After temporal integration, the signal-to-noise ratio of the device will decrease if not correctly aligned with the vessels, therefore indicating non-adherence, and on the contrary, the signal-to-noise ratio will remain on image parts with the contrast spatially computed in the reference image if the lead adheres to the vessel wall.

In general, the signal-to-noise will not increase when the lead is not adhered to vessel wall.
- In an option, an analysis is computed to detect areas more or less adherent, with thresholding operations based on the adherence level, such as percentage of contrast decrease, and on the extent of the adherence versus non-adherence areas.

As a result, an output on lead adhesion is provided after a venogram is captured. This can be arranged during a lead extraction procedure.

An example for a field of application is X-ray guided lead extraction procedures.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for determining an adhesion of a lead inside a vessel.
Fig. 2 shows an example of a system for determining an adhesion of a lead inside a vessel in the context of an operation room.
Fig. 3 shows basic steps of an example of a method for determining an adhesion of a lead inside a vessel.
Fig. 4 shows an example of an illustration of a venogram with an identified potential adhesion of a lead inside a vessel.
Fig. 5 shows an example of a further workflow for determining an adhesion of a lead inside a vessel.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

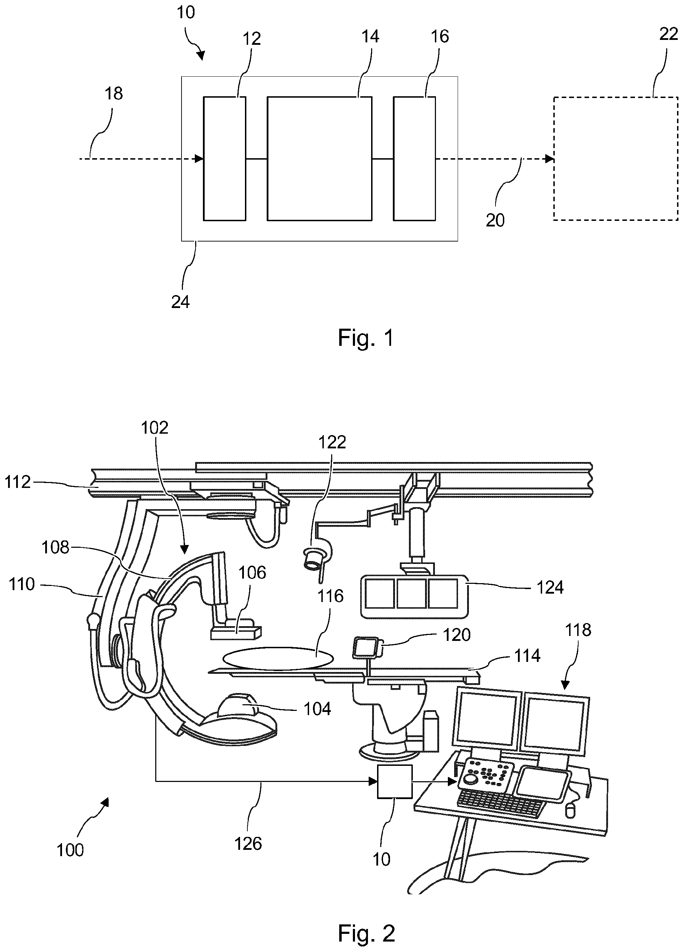

Fig. 1 schematically shows an example of a device 10 for determining an adhesion of a lead inside a vessel. The device 10 comprises a data input 12, a data processor 14 and an output interface 16. The data input 12 is configured to provide a plurality of images of a region of interest of a blood vessel of a subject, in which region of interest a lead is located inside the vessel. The data processor 14 is configured to align at least a part of the images of the plurality of images with each other. The aligning is provided based on a predetermined feature in the image relating to a spatial relation of the lead in the vessel. The data processor 14 is also configured to stack the aligned images. The output interface 16 is configured to provide the stacked images for assessment of an adhesion of the lead at an inner side of the vessel wall.

In Fig. 1, a first arrow 18 in broken lines indicates data supplied to the device 10 for determining the adhesion of a lead inside a vessel. A second arrow 20 in broken lines indicates the data provided by the output interfacecl6, i.e. the stacked images.

The term "lead" relates to e.g. a small wire of an implant, such as a pacemaker. As an example the wire is running along a part of the vessel.

The term "plurality of images" relates to at least two images, or more than two images. For an assessment of moving and non-moving parts, a larger number of images is provided. In an example, a sequence of images is provided. In an option, a livestream of images is provided.

The term "to align" relates to arranging the plurality of images such that the predetermined feature of a subsequent image matches the predetermined feature of the previous image. Other parts may be non-aligned.

The term "to stack" relates to arranging the plurality of images on top of each other, or below each other, in s staple like manner. The user can then "see through" all stacked images, i.e. the user is provided as if he/she is looking through the images.

The term "predetermined feature" relates to a feature that is expected to be visible in the plurality of images. For example, the predetermined feature is a part of the vessel wall or a part of the lead.

The term "spatial relation" relates to a relative arrangement of the vessel wall and the lead. In an example, a non-adhered lead is moving within the vessel due to heartbeat motion resulting in blood flow in the vessel. In another example, an adhered lead is not moving with respect to the vessel, despite the heartbeat motion resulting in blood flow in the vessel. The lead may be abutting the wall such that it is at least partly embedded in the tissue of the wall structure.

The term "adhesion" relates to a lead section that is removably fixed to the tissue, such as being embedded in tissue structure.

The term "data input" relates to providing or supplying data for data processing steps. The data input 12 can also be referred to as image data input. The data input 12 can also be referred to as data supply, as image data supply, as image input, as input unit or simply as input. In an example, the image data input 12 is data-connectable to an imaging source arrangement.

The term "data processor" relates to a processor or part of a processor arrangement that is provided to conduct the computing steps using the data supplied by the data input 12. The data processor 14 can also be referred to as data processing arrangement, as processor unit or as processor. In an example, the data processor 14 is data-connected to the data input 12 and the output interface 16.

The term "output interface" relates to an interface for providing the processed or computed data for further purposes. The output interface 16 can also be referred to as output or output unit. In an example, the output interface 16 is data-connectable to a display arrangement or display device. In another example, the output is data-connected to a display. As an example, the signals by the controller can be provided by the output interface 16.

In an option of the device of Fig. 1, after the alignment, an increase of a signal-to-noise ratio for parts of the image other than the predetermined feature indicates a static relation of the respective part of the image and the predetermined feature.

The term "static relation" relates to a relative non-movement. Of course, due to heartbeat, a motion of the vessel or vessel wall in relation to the subject's surrounding body structure can be provided.

In another option of the device of Fig. 1, for the alignment, the data processor 14 is configured to segment the lead in at least one image. For the alignment, the data processor 14 is configured to identify an edge of the vessel in at least one image. After the stacking, the data processor 14 is configured to search the images for markers to determine relative movement.

The term "segmented" relates to identifying the lead within the image based on image content.

The term "markers" relates to identifiable parts like a bent portion, contour changes or actual X-ray markers on the lead.

As an example, contrast is injected, and the vessel is identified based on contrast images.

As an example, contrast is only partly injected, still allowing to identify the lead within the contrasted vasculature.

In a first optional variation of the device of Fig. 1, the data processor 14 is configured to provide the aligning with respect to a wall section of the vessel. As an option, the wall section of the vessel for alignment is in a region of the vessel around the lead. As an option, after the alignment, an increase of a signal-to-noise ratio for a part of the lead indicates a static relation of the lead and the vessel wall.

The term "wall section" relates to a part of the vessel wall.

The alignment combined with the periodic movement of the lead inside the vessel due to heartbeat motion smears out other stuff and focusses on the spatial relation of the lead and the vessel.

An increase of a signal-to-noise ratio for the part of the lead indicates an adhesion of the lead to the vessel wall. An increase of the signal-to-noise ratio for the part of the lead indicates a static structure of the lead and the vessel wall.

In an example, the images are stacked with respect to the vessel wall. As a result, the signal-to-noise of the vessel wall increases after stacking. For each part of the lead, the increase in signal-to-noise is determined. For those parts where the signal-to-noise does not increase, this is an indication that the lead is not attached to the vessel wall.

In a second optional variation of the example of Fig. 1, the data processor 14 is configured to provide the aligning with respect to a section of the lead in the region of interest. As an option, the section of the lead is in a region of the vessel where the lead is closer to the vessel wall. As an option, after the alignment, an increase of a signal-to-noise ratio for a part of the vessel wall adjacent to the lead indicates a static relation of the vessel wall and the lead.

In an example, the first optional variation and the second optional variation are provided as alternative approaches. For example, only one is applied.

In another example, the first optional variation and the second optional variation are provided as alternative approaches, applied in an alternating manner for achieving two different assessments.

In a further example, the first optional variation and the second optional variation are provided as alternative approaches, applied for the same sequence as parallel, but independent procedures, for achieving two different assessments.

The term "section of the lead" relates to a part or portion of the lead.

An increase of a signal-to-noise ratio for the part of the vessel wall indicates an adhesion of the lead to the vessel wall. An increase of the signal-to-noise ratio for the part of the vessel wall indicates a static structure of the lead and the vessel wall.

In an option of the example of Fig. 1, the data processor 14 is configured to mark image parts indicating the static relation, after the stacking.

The term "marked" relates to visually highlighting the portion where the static relation has been detected.

In Fig. 1, the second arrow 20 in broken lines my also indicate the image data provided, for example, to a display, indicated with a broken line frame 22.

Further in Fig. 1, a further frame 24 indicates an option according to which the data input 12, the data processor 14 and the output interface 16 are arranged in a common housing, e.g. in an integrated manner. However, in an option, they are provided in a separated arrangement.

In an option of the example of Fig. 1, for the assessment of the adhesion, the data processor 14 is configured to identify a signal-to-noise ratio (S/N or SNR). As an option, for the assessment of the adhesion, the data processor 14 is configured to i) identify lead sections in the images and to assess a respective signal-to-noise ratio for the lead sections. Alternatively, or in addition, for the assessment of the adhesion, the data processor 14 is configured to ii) identify vessel sections in the images and to assess a respective signal-to-noise ratio for the vessel sections.

In an example, the images are venograms.

In an option of the example of Fig. 1, the data processor 14 is configured to identify, as a base, a signal-to-noise ratio for a first part of the vessel. The data processor 14 is configured to identify a signal-to-noise ratio for further parts of the vessel that are linked to the lead inside the vessel. For the identification of the signal-to-noise ratio, the first part acts as a base.

The image of the vessel comprises at least a number of the parts of the vessel.

The term "connected" relates to parts where the lead is not free moving, but adhered to the wall.

The term "non-connected" relates to parts where the lead is free moving inside the vessel.

In an option of the example of Fig. 1, the data input 12 is configured to provide a predetermined threshold of the signal-to-noise ratio to differentiate moving and non-moving parts of the lead. The data processor 14 is configured to label the non-moving parts as adhered lead portions.

The term "to label" relates to indicating or marking the respective part.

In an example, the data processor is configured to apply a threshold to identify connected and non-connected parts of the lead. The output interface 16 is configured to provide an image of the vessel to a user. At least one of the group of the connected and the non-connected parts are indicated within the image. For example, both groups are indicated.

In an option of the example of Fig. 1, for the aligning, the data processor 14 is configured to warp at least some of the images for an improved alignment.

The term "to warp" relates to an elastic registration, in which a deformation of the image, or at least a part of the image, is provided for a better matching, i.e. for a better alignment.

In an example, a rigid registration is performed for alignment.

In another example, an elastic registration is performed for alignment.

In a further example, a rigid and an elastic registration are performed for alignment.

In another example, a rigid registration is provided in combination with a sliding window triggering a repeated aligning and stacking.

In an option of the example of Fig. 1, the data processor 14 is configured to provide the aligning and the stacking for a selection-part of the vessel. The selection-part is movable along the vessel to provide a repeated aligning and stacking along the vessel structure.

The term "selection-part" relates to a portion, instead of the complete region of interest.

The term "repeated" relates to a continuous, e.g. stepwise procedure.

A sliding window is provided for providing an adhesion assessment along the vessel and along the lead. The signal-to-noise ratio is determined along the vessel.

In an option of the example of Fig. 1, the images are X-ray images. As an option, at least a part of the images shows an at least partly contrast-injected vasculature.

Fig. 2 shows an example of a system 100 for determining an adhesion of a lead inside a vessel in the context of an operation room. The system 100 comprises an imaging device 102 and an example of the device 10 for determining an adhesion of a lead inside a vessel according to one of the preceding examples. The imaging device 102 is configured to provide the plurality of images of region of interest of a blood vessel of a subject.

As an example, the imaging device 102 is an X-ray imaging device with an X-ray source 104 and an X-ray detector 106 mounted to opposite ends of a C-arm structure 108 that is movably hold by a support arm 110 that is connected to a ceiling mounted rail system 112. A subject support 114 is provided with a subject 116 arranged on top. A console 118 is shown in the foreground (bottom right), with input and display devices like monitors, keyboard, trackpad, mouse, control knobs and others.

Further, a bedside 120 controller interface may be provided, as well as additional lighting 122 and a display arrangement 124.

A connection line 126 indicates the data connection (e.g. wireless or wire-bound) between the imaging device 102 and the device 10 for determining an adhesion of a lead inside a vessel.

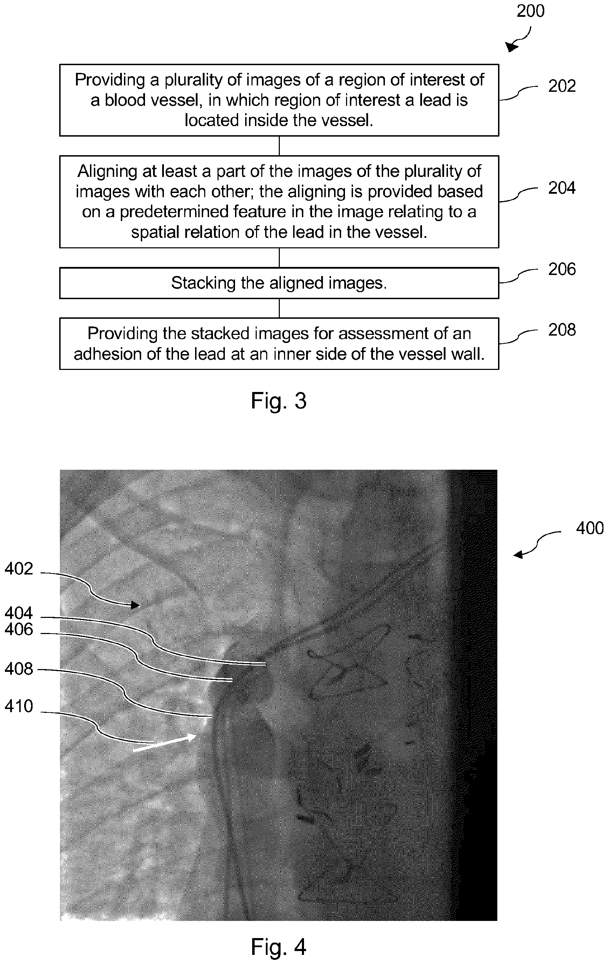

Fig. 3 shows basic steps of an example of a method 200 for determining an adhesion of a lead inside a vessel. The method comprises the following steps:
- In a first step 202, a plurality of images is provided of a region of interest of a blood vessel, in which region of interest a lead is located inside the vessel.
- In a second step 204, at least a part of the images of the plurality of images is aligned with each other. The aligning is provided based on a predetermined feature in the image relating to a spatial relation of the lead in the vessel.
- In a third step 206, the aligned images are stacked.
- In a fourth step 208, the stacked images are provided for assessment of an adhesion of the lead at an inner side of the vessel wall.

In an example of the method, after the alignment, an increase of a signal-to-noise ratio for parts of the image other than the predetermined feature indicates a static relation of the respective part of the image and the predetermined feature.

In an example of the method, for the alignment, the lead is segmented in at least one image. For the alignment, an edge of the vessel is identified in at least one image. After the stacking, the images are searched for markers to determine relative movement.

In an example of the method, the aligning is provided with respect to a wall section of the vessel. In an option, the wall section of the vessel for alignment is in a region of the vessel around the lead. In an option, after the alignment, an increase of a signal-to-noise ratio for a part of the lead indicates a static relation of the lead and the vessel wall.

In an example of the method, the aligning is provided with respect to a section of the lead in the region of interest. In an option, the section of the lead is in a region of the vessel where the lead is closer to the vessel wall. In an option, after the alignment, an increase of a signal-to-noise ratio for a part of the vessel wall adjacent to the lead indicates a static relation of the vessel wall and the lead.

In an example of the method, after the stacking, image parts indicating the static relation are marked.

In an example of the method, for the assessment of the adhesion, a signal-to-noise ratio is identified. In an option, for the assessment of the adhesion, lead sections in the images are identified. In a further option, in addition or alternatively, vessel sections in the images are identified.

In an example of the method, as a base, a signal-to-noise ratio is identified for a first part of the vessel. A signal-to-noise ratio is identified for further parts of the vessel that are linked to the lead inside the vessel. For the identification of the signal-to-noise ratio, the first part acts as a base. A threshold is applied to identify connected and non-connected parts of the lead. An image of the vessel is provided and wherein at least one of the group of the connected and the non-connected parts are indicated within the image.

In an example of the method, a predetermined threshold of the signal-to-noise ratio is provided to differentiate moving and non-moving parts of the lead; the non-moving parts are labelled as adhered lead portions.

In an example of the method, for the aligning, at least some of the images are warped for an improved alignment.

In an example of the method, the aligning and stacking is provided for a selection-part of the vessel. The selection-part is movable along the vessel to provide a repeated aligning and stacking along the vessel structure.

In an example of the method, the images are X-ray images. In an option, at least a part of the images shows an at least partly contrast-injected vasculature.

Fig. 4 shows an example of an illustration of an X-ray image 400 showing an anatomic structure 402. Besides bone structure, also a vessel 404 is shown, with a lead 406 inserted within the vessel 404. Further, a potential adhesion 408 of the lead 406 inside the vessel 404 is determined based on the image processing steps provided above. An arrow 410 points to the potential adhesion 408 of the lead 406 inside the vessel 404.

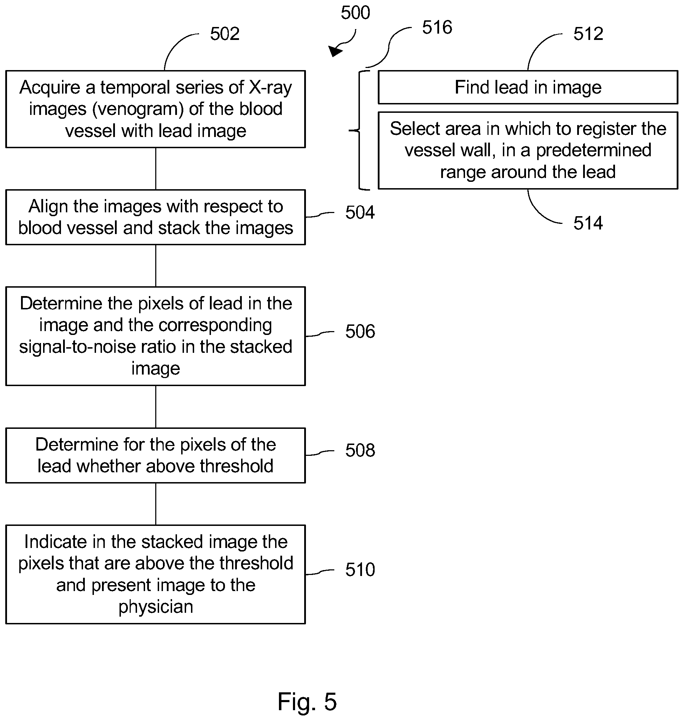

Fig. 5 shows an example of a further workflow 500 for determining an adhesion of a lead inside a vessel.

In a first frame 502, a temporal series of X-ray images (venogram) of the blood vessel with lead image is acquired.

In a second frame 504, the images are aligned with respect to blood vessel. Further, the images are stacked.

In a third frame 506, the pixels of lead are determined, i.e. identified, in the image and the corresponding signal-to-noise ratio in the stacked image is determined.

In a fourth frame 508, it is determined for the pixels of the lead whether the signal-to-noise ratio is above a threshold.

In a fifth frame 510, the pixels are indicated in the stacked image that are above the threshold and this is presented as an image to the physician.

In an option, as a first additional step it is provided to find the lead in the images. As a second additional step, an area is selected in which to register the vessel wall, e.g. in a predetermined range around the lead.

The term "subject" may also be referred to as individual. The "subject" may further also be referred to as patient, although it is noted that this term does not indicate whether any illness or disease is actually present with the subject.

In an example, a computer program or program element for controlling an apparatus according to one of the examples above is provided, which program or program element, when being executed by a processing unit, is adapted to perform the method steps of one of the method examples above.

In an example, a computer readable medium having stored the computer program of the preceding example is provided.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for determining an adhesion of a lead inside a vessel, the device comprising:
- a data input (12);
- a data processor (14); and
- an output interface (16);
wherein the data input is configured to provide a plurality of images of a region of interest of a blood vessel of a subject, in which region of interest a lead is located inside the vessel;
wherein the data processor is configured to align at least a part of the images of the plurality of images with each other; wherein the aligning is provided based on a predetermined feature in the image relating to a spatial relation of the lead in the vessel; and to stack the aligned images; and
wherein the output interface is configured to provide the stacked images for assessment of an adhesion of the lead at an inner side of the vessel wall.

2. Device according to claim 1, wherein, after the alignment, an increase of a signal-to-noise ratio for parts of the image other than the predetermined feature indicates a static relation of the respective part of the image and the predetermined feature.

3. Device according to claim 1 or 2, wherein, for the alignment, the data processor is configured to segment the lead in at least one image;
wherein, for the alignment, the data processor is configured to identify an edge of the vessel in at least one image; and
wherein, after the stacking, the data processor is configured to search the images for markers to determine relative movement.

4. Device according to claim 1, 2 or 3, wherein the data processor is configured to provide the aligning with respect to a wall section of the vessel;
wherein the wall section of the vessel for alignment is in a region of the vessel around the lead; and
wherein after the alignment, an increase of a signal-to-noise ratio for a part of the lead indicates a static relation of the lead and the vessel wall.

5. Device according to claim 1, 2 or 3, wherein the data processor is configured to provide the aligning with respect to a section of the lead in the region of interest;
wherein the section of the lead is in a region of the vessel where the lead is closer to the vessel wall; and
wherein after the alignment, an increase of a signal-to-noise ratio for a part of the vessel wall adjacent to the lead indicates a static relation of the vessel wall and the lead.

6. Device according to one of the preceding claims, wherein the data processor is configured to mark image parts indicating the static relation, after the stacking.

7. Device according to one of the preceding claims, wherein, for the assessment of the adhesion, the data processor is configured to identify a signal-to-noise ratio; and
wherein for the assessment of the adhesion, the data processor is configured:
i) to identify lead sections in the images and to assess a respective signal-to-noise ratio for the lead sections; and/or
ii) to identify vessel sections in the images and to assess a respective signal-to-noise ratio for the vessel sections.

8. Device according to one of the preceding claims, wherein the data processor is configured to identify, as a base, a signal-to-noise ratio for a first part of the vessel;
wherein the data processor is configured to identify a signal-to-noise ratio for further parts of the vessel that are linked to the lead inside the vessel; and
wherein for the identification of the signal-to-noise ratio, the first part acts as a base.

9. Device according to one of the preceding claims, wherein the data input is configured to provide a predetermined threshold of the signal-to-noise ratio to differentiate moving and non-moving parts of the lead; and
wherein the data processor is configured to label the non-moving parts as adhered lead portions.

10. Device according to one of the preceding claims, wherein, for the aligning, the data processor is configured to warp at least some of the images for an improved alignment.

11. Device according to one of the preceding claims, wherein the data processor is configured to provide the aligning and the stacking for a selection-part of the vessel; and
wherein the selection-part is movable along the vessel to provide a repeated aligning and stacking along the vessel structure.

12. Device according to one of the preceding claims, wherein the images are X-ray images; and
wherein at least a part of the images shows an at least partly contrast-injected vasculature.

13. A system (100) for determining an adhesion of a lead inside a vessel, the system comprising:
- an imaging device (102); and
- a device (10) for determining an adhesion of a lead inside a vessel according to one of the preceding claims,
wherein the imaging device is configured to provide the plurality of images of region of interest of a blood vessel of a subject.

14. A method (200) for determining an adhesion of a lead inside a vessel, the method comprising the following steps:
- providing (202) a plurality of images of a region of interest of a blood vessel, in which region of interest a lead is located inside the vessel;
- aligning (204) at least a part of the images of the plurality of images with each other; wherein the aligning is provided based on a predetermined feature in the image relating to a spatial relation of the lead in the vessel;
- stacking (206) the aligned images; and
- providing (208) the stacked images for assessment of an adhesion of the lead at an inner side of the vessel wall.

15. Computer program comprising instructions which, when the program is executed by a computer, cause the computer to carry out the method of claim 14.
